## Europäisches Patentamt

## European Patent Office

## Office européen des brevets

(19)

(11) Veröffentlichungsnummer: **0 061 018**
**B1**

(12) **EUROPÄISCHE PATENTSCHRIFT**

(45) Veröffentlichungstag der Patentschrift:
**31.10.84**

(21) Anmeldenummer: **82101479.2**

(22) Anmeldetag: **26.02.82**

(51) Int. Cl.³: **B 01 J 23/92, B 01 J 27/32,
B 01 J 37/20, B 01 J 37/28,
C 07 C 51/265**

(54) **Verfahren zur Reaktivierung von Vanadium enthaltenden Oxidationskatalysatoren.**

(30) Priorität: **04.03.81 DE 3108101**

(43) Veröffentlichungstag der Anmeldung:
**29.09.82 Patentblatt 82/39**

(45) Bekanntmachung des Hinweises auf die Patenterteilung:
**31.10.84 Patentblatt 84/44**

(84) Benannte Vertragsstaaten:
**AT BE DE FR GB IT**

(56) Entgegenhaltungen:
**FR - A - 2 328 510**
**GB - A - 1 464 198**
**US - A - 3 474 041**
**US - A - 4 123 442**

(73) Patentinhaber: **BASF Aktiengesellschaft,
Carl-Bosch-Strasse 38, D-6700 Ludwigshafen (DE)**

(72) Erfinder: **Reuter, Peter, Dr., Neuwiesenstrasse 22,
D-6700 Ludwigshafen (DE)**
Erfinder: **Blechschmitt, Kurt, Finkenstrasse 15,
D-6707 Schifferstadt (DE)**
Erfinder: **Wirth, Friedrich, Dr., Schlossgasse 6,
D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft ein Verfahren zur Reaktivierung von Vanadium enthaltenden Trägerkatalysatoren, die für die Oxidation von o-Xylol oder Naphthalin zu Phthalsäureanhydrid verwendet werden.

Katalysatoren, die Vanadium enthalten, lassen sich bekanntlich für die Herstellung von Carbonsäuren, Carbonsäurenanhydriden oder Chinonen durch Oxidation von gesättigten oder ungesättigten aliphatischen oder aromatischen Kohlenwasserstoffen verwenden. Technisch führt man die Oxidation in der Dampfphase mit Luft in Festbett- oder Wirbelbettreaktoren durch.

Großtechnische Bedeutung haben z. B. solche Vanadium enthaltende Katalysatoren erlangt, die für die Herstellung von Phthalsäureanhydrid (PSA) aus o-Xylol oder Naphthalin, Maleinsäureanhydrid (MSA) aus Benzol oder gesättigten oder ungesättigten aliphatischen Kohlenwasserstoffen mit 4 bis 6 Kohlenstoffatomen oder von Antrachinon aus Diphenylmethanverbindungen herangezogen werden. Katalysatoren dieser Art, die das Vanadium z. B. in Form von Vanadiumpentoxid neben Titandioxid enthalten und die in ihrer aktiven Masse noch andere wirksame Bestandteile, wie die Oxide der Elemente Caesium, Rubidium, Phosphor oder Antimon aufweisen, werden z. B. in der DE-A-2 436 009 oder der DE-A-2 510 994 beschrieben.

Diese Vanadium enthaltenden Katalysatoren haben den Nachteil, daß sie während des Betriebs ständig an Aktivität verlieren. Um einen ausreichenden Umsatz aufrechtzuerhalten, müssen deshalb die Reaktionsbedingungen fortwährend verschärft werden. Dies kann z. B. durch Erhöhung der Reaktionstemperatur, durch Erniedrigung des Luftdurchsatzes oder durch Zurücknahme der Beladung der Luft bzw. des Trägergases mit dem zu oxidierenden Kohlenwasserstoff geschehen.

Der damit verbundene Ausbeuteverlust bzw. der Rückgang an Kapazität wird dabei schließlich so groß, daß der Katalysator nicht mehr wirtschaftlich betrieben werden kann, so daß man dazu gezwungen wird, den Katalysator auszuwechseln. Ein Katalysatorwechsel ist sehr aufwendig, da die Anlage für Wochen abgestellt werden muß, was mit einem Produktionsausfall verbunden ist. Außerdem sind die Katalysatoren teuer.

Man hat deshalb schon versucht, der Desaktivierung der Katalysatoren durch eine laufende Zugabe von aktivierend wirkenden Stoffen zum Katalysator entgegenzuwirken. So gibt man z. B. bei der Oxidation von o-Xylol zu PSA Schwefeldioxid hinzu. Ohne eine ständige Zugabe dieser Aktivierungsstoffe verliert der Katalysator noch rascher an Aktivität und die Betriebsbedingungen müssen zur Aufrechterhaltung der erwünschten Ergebnisse schnell verschärft werden. Da die Aktivierungsstoffe, wie $SO_2$ die Anlage ganz oder teilweise unverändert verlassen, belasten sie die Umwelt. Ihre Entfernung aus den die Anlage verlassenden Abgasen ist sehr aufwendig.

Es war daher die Aufgabe gestellt, ein Verfahren zur Reaktivierung von ganz oder teilweise desaktivierten Vanadium enthaltenden Katalysatoren zu finden, das die fortwährende Zugabe von Aktivierungsstoffen während des Betriebs der Katalysatoren überflüssig macht und die Aufrechterhaltung hoher Ausbeuten und Durchsätze bei der katalytischen Oxidation ermöglicht.

Nach dem erfindungsgemäßen Verfahren, das diese Forderungen erfüllt, wird die Reaktivierung von Vanadium enthaltenden Trägerkatalysatoren, die für die Oxidation von o-Xylol oder Naphthalin zu Phthalsäureanhydrid verwendet werden, so vorgenommen, daß man den Trägerkatalysator, der aus einem inerten Träger und der darauf aufgebrachten aktiven Masse besteht, wobei die aktive Masse 1 bis 30 Gew.-% Vanadiumpentoxid, 70 bis 99 Gew.-% Titanoxid und bis zu 5 Gew.-% Oxide der Elemente Rubidium, Caesium, Phosphor oder Antimon enthält, zuerst bei Temperaturen von 100 bis 600°C in Gegenwart eines Sauerstoff enthaltenden Gases mit einer flüchtigen Phosphorverbindung und dann bei Temperaturen von 50 bis 600°C mit Schwefeltrioxid oder einem Gemisch aus einer flüchtigen Schwefelverbindung und einem Sauerstoff enthaltenden Gas behandelt.

Es ist zwar aus der DE-B-1 468 816 bekannt, daß man Oxidationskatalysatoren, die Vanadium und Phosphor enthalten, durch Zugabe von Phosphiten reaktivieren kann, und in der US-A-4 123 442 wird beschrieben, daß man derartige Katalysatoren, die für die Herstellung von Maleinsäureanhydrid verwendet werden, zur Reaktivierung mit Schwefeltrioxid behandelt. Das erfindungsgemäße Verfahren ist durch diese Arbeitsweisen jedoch nicht nahegelegt, da die vorteilhaften Ergebnisse des Verfahrens der Erfindung nicht erhalten werden, wenn man den Katalysator allein mit der Phosphorverbindung oder der Schwefelverbindung behandelt oder wenn man die Behandlungsmittel in umgekehrter Reihenfolge anwendet.

Als flüchtige Phosphorverbindung kommen solche Verbindungen in Betracht, die bei den Temperaturen der erfindungsgemäßen Reaktivierung flüchtig sind. Geeignete Phosphorverbindungen sind z. B. Phosphine, wie Trimethylphosphin, Phosphinoxide, wie Trimethylphosphinoxid, Phosphorwasserstoff, Phosphoroxide, wie Phosphorpentoxid, Ester der Phosphor- oder der phosphorigen Säure, wie $OP(O[CH_2]_3 CH_3)_3$, Phosphorhalogenide, wie Phosphortrichlorid, Phosphoroxidhalogenide oder Phosphorsulfohalogenide wie Phosphoroxichlorid.

Neben Schwefeltrioxid kommen als flüchtige Schwefelverbindungen bevorzugt solche Verbindungen in Betracht, die sich unter den Reaktivierungsbedingungen mit Luft oder einem anderen sauerstoffhaltigen Gas am Katalysator ganz oder teilweise in Schwefeltrioxid umsetzen, wie Schwefeldioxid, Schwefelwasserstoff, Mercaptane, wie Äthylmercaptan, Schwefelkohlenstoff, elementarer Schwefel oder Schwefelhalogenide, wie Schwefeldichlorid, Schwefeloxihalogenide, wie Thionylchlorid.

Besonders gute Ergebnisse werden erhalten, wenn man Triphenylphosphin als Phosphorverbindung und $SO_3$ bzw. $SO_2$ als Schwefelverbindung verwendet.

Das erfindungsgemäße Verfahren eignet sich für die Reaktivierung vanadiumhaltiger Trägerkatalysatoren, die z. B. in der DE-A-2 510 994 beschrieben werden. Katalysatoren dieser Art werden zur Erzeugung von PSA bekanntlich in einem Röhrenofen, dessen Rohre einen Durchmesser von 18 bis 40 mm und eine Länge von 1,0 bis 4,0 m haben, angeordnet, wobei die Rohre zur Temperaturregelung von einer Salzschmelze umgeben sind, in der man eine Temperatur von 340 bis 450° C enthält. Durch den Röhrenofen leitet man das gasförmige Gemisch aus Luft und dem zu oxidierenden Kohlenwasserstoff.

Das neue Reaktivierungsverfahren wird z. B. so durchgeführt, daß man die Phosphorverbindung der Luft oder einem anderen sauerstoffhaltigen Gas, welche auf Temperaturen von 20 bis 500° C, vorteilhaft 100 bis 300° C, vorerhitzt sind, zugibt und das Gemisch über den in einen Röhrenreaktor angeordneten Katalysator leitet. Der Katalysator wird durch das die Reaktorrohre umströmende Wärmeübertragungsmedium auf eine Temperatur von 100 bis 600° C, vorteilhaft 200 bis 500° C, erhitzt. Die Menge an Phosphorverbindung wird so bemessen, daß pro 100 g katalytischer Masse im Katalysator 1,0 bis 10 000, vorteilhaft 10 bis 1000 mg Phosphor eingesetzt werden. Die Menge an der mit der Phosphorverbindung beladenen Luft oder die Trägergasmenge beträgt pro 100 g katalytische Masse stündlich 0,01 bis 8,0 $Nm^3$, vorteilhaft 0,1 bis 5,0 $Nm^3$. Das Gemisch aus der Phosphorverbindung und der Luft oder dem Trägergas hat einen Phosphorgehalt von 0,01 bis 10 000 mg, vorteilhaft 0,1 bis 1000 mg.

Nach einer besonders vorteilhaften Ausführungsform der Erfindung gibt man die Phosphorverbindung zur Aktivierung des Katalysators während des Betriebes, d. h. während der Oxidation von z. B. o-Xylol mit Luft hinzu, da dadurch die Produktion an PSA nicht unterbrochen werden muß und eine besonders schnelle und gute Aktivierung erhalten wird. Dazu wird die Phosphorverbindung dem Trägergas vor dem Einleiten in den Reaktor flüssig oder gasförmig zugegeben. Man kann die Phosphorverbindung aber auch in flüssigem o-Xylol lösen und sie mit dem o-Xylol der Luft zugeben.

Die Menge an Luft bzw. Trägergas pro 100 g katalytischer Masse beträgt dabei 0,5 bis 8,0, vorteilhaft 1,0 bis 6 $Nm^3/h$, die Konzentration an o-Xylol im Trägergas 20 bis 150 $g/Nm^3$ und die Konzentration an Phosphor in Form der Phosphorverbindungen im Trägergas 0,01 bis 10 000 mg, vorteilhaft 0,1 bis 1000 $mg/Nm^3$. Die Menge an Phosphor pro 100 g katalytischem Material beträgt 1,0 bis 10 000 mg, vorteilhaft 10 bis 1000 mg.

Die Temperatur der Salzschmelze, die die Temperatur des Reaktors regelt, beträgt 300 bis 500° C, vorteilhaft 340 bis 450° C. Das Sauerstoff enthaltende Trägergas wird vor der Zugabe von o-Xylol und der Phosphorverbindung auf 60 bis 500° C, vorteilhaft 100 bis 300° C erhitzt.

Die Einwirkungsdauer der Phosphorverbindung, bzw. die Menge derselben, die auf den Katalysator einwirkt, richtet sich nach der restlichen Aktivität des Katalysators und ist von Fall zu Fall verschieden.

Nach der Behandlung des Katalysators mit der Phosphorverbindung ist noch keine Aktivierung des Katalysators festzustellen. Der Katalysator hat häufig durch die Phosphorbehandlung noch weiter an Aktivität verloren, was sich daran zeigt, daß bei der Umsetzung von o-Xylol mit Luft oder dem Sauerstoff enthaltenden Trägergas die Temperatur des Salzes angehoben werden muß.

Erst nach oder während der anschließenden Behandlung des Katalysators mit der Schwefelverbindung wird eine merkliche und bleibende Aktivierung erhalten.

Bei der anschließenden Behandlung des Katalysators mit der Schwefelverbindung kann in unterschiedlicher Weise verfahren werden. Wird $SO_3$ angewendet, so kann dieses alleine, im Gemisch mit Luft bzw. dem Trägergas oder auch im Gemisch mit Luft bzw. dem Trägergas und o-Xylol eingesetzt werden. Wird $SO_2$ oder eine andere flüchtige Schwefelverbindung eingesetzt wird diese zusammen mit dem Sauerstoff enthaltenden Trägergas, gegebenenfalls im Gemisch mit o-Xylol eingesetzt.

Bei der Behandlung des Katalysators mit $SO_3$, mit $SO_3$ im Gemisch mit Luft oder dem Trägergas, mit $SO_2$ oder einer anderen Schwefelverbindung im Gemisch mit dem sauerstoffhaltigen Trägergas, werden diese Stoffe oder Sauerstoffgemische über den Katalysator geleitet oder der Katalysator wird unter diese Stoffe oder Stoffgemische gestellt. Der Katalysator sollte dabei eine Temperatur von 50 bis 600° C, vorteilhaft von 300 bis 500° C, aufweisen.

Wird $SO_3$ im Gemisch mit einem Trägergas angewendet, so muß dieses nicht sauerstoffhaltig sein. Sauerstofffreie Trägergase sind z. B. Stickstoff oder Kohlendioxid.

Wird $SO_3$, $SO_2$ bzw. eine andere Schwefelverbindung im Gemisch mit Luft bzw. Trägergas über den Katalysator geleitet, werden von diesen Gemischen stündlich 0,1 bis 8,0, vorteilhaft 1,0 bis 5,0 $Nm^3$ pro 100 g katalytischer Masse angewandt.

Werden die Schwefelverbindungen im Gemisch mit dem sauerstoffhaltigen Trägergas eingesetzt, so kann das Trägergas mit o-Xylol beladen sein. Die Temperatur der Salzschmelze zur Temperaturregelung beträgt in diesem Falle 300 bis 500° C, vorteilhaft 340 bis 450° C.

Die Menge an Gasgemisch, welches über den Katalysator geleitet wird, beträgt 0,5 bis 8,0, vorteilhaft 1,0 bis 6 $Nm^3/h$. Die Konzentration an o-Xylol pro Nm³ Gasgemisch kann bis zu 150 g betragen.

Die Konzentration von $SO_3$, $SO_2$ bzw. einer geeigneten Schwefelverbindung in der Luft bzw. in dem Trägergas, die auch mit o-Xylol beladen sein kann, kann in weiten Grenzen variiert werden. Zweckmäßigerweise arbeitet man mit einem Überschuß an Luft bzw. Trägergas, besonders wenn dieses mit o-Xylol beladen ist.

Die zur Aktivierung des Katalysators nötige Menge an $SO_3$, $SO_2$ oder einer anderen Schwefelverbindung, ist von Fall zu Fall verschieden und hängt u. a. von dem Desaktivierungsgrad des Katalysators vor der Aktivierung ab. Diese Menge kann experimentiell bestimmt werden, indem die Aktivität des Katalysators während oder nach der Aktivierung bei der Oxidation von o-Xylol zu PSA bestimmt wird. Eine mehrmalige Behandlung mit $SO_3$, $SO_2$ oder einer Schwefelverbindung kann notwendig sein, bis eine Steigerung der Aktivität des Katalysators nicht mehr beobachtet wird.

Die Schwefelverbindungen werden der Luft oder dem Trägergas flüssig oder gasförmig zugegeben. Wenn sich die Schwefelverbindungen in o-Xylol lösen oder sich mit diesem mischen, werden sie vorteilhaft zusammen mit dem o-Xylol der Luft oder dem Trägergas zugegeben.

Beispiel

a) Herstellung von Phthalsäureanhydrid (PSA) durch Luftoxidation von o-Xylol an einem Vanadium enthaltenden Trägerkatalysator

Durch einen Reaktor von 3,25 m Länge und 25 mm lichter Weite, der zur Temperaturregelung von einer Salzschmelze umströmt wird, leitet man stündlich von oben nach unten 4,0 Nm³ Luft mit Beladungen an 97 Gew.-% o-Xylol von 60 g/Nm³. Die Temperatur der Salzschmelze beträgt 374°C. Die Reaktionsgase werden abgekühlt, wobei sich das gebildete PSA abscheidet.

Der Reaktor ist auf einer Länge von 2,80 m mit zwei verschiedenen Katalysatoren gefüllt, wobei der erste Katalysator in Strömungsrichtung 1,20 m und der zweite Katalysator 1,60 m des Rohrreaktors einnimmt. Der erste Katalysator besteht aus einem inerten Träger, der mit einer katalytisch wirksamen Masse aus 7 Gew.-% Vanadiumpentoxid, 0,13 Gew.-% Rubidium in Form von Rubiudiumoxid und 92,87 Gew.-% Titandioxid in Form von Anatas beschichtet ist. Der zweite Katalysator besteht aus einem inerten Träger, der mit einer katalytisch wirksamen Masse aus 7 Gew.-% Vanadiumpentoxid, 0,5 Gew.-% Phosphor in Form von Phosphorpentoxid und 92,5 Gew.-% Titandioxid in Form von Anatas beschichtet ist. Der Gehalt an katalytischer Masse der in den Reaktor eingefüllten Katalysatoren beträgt insgesamt 116 g.

Nach einer Betriebszeit von 300 Tagen erhält man eine Ausbeute an PSA von 112 Gew.-% (berechnet auf 100%iges o-Xylol). Der Gehalt an Phthalid im erzeugten PSA beträgt 0,01 Gew.-%.

Nach einer Laufzeit von 460 Tagen ist es erforderlich, die Temperatur der Salzschmelze auf 394°C anzuheben, um den Phthalidgehalt im erzeugten PSA unter 0,03% zu halten. Die Ausbeute an PSA ist auf 104 Gew.-% gefallen.

b) Reaktivierung des Katalysators

Nach 461tägiger Laufzeit der Oxidation von o-Xylol zu PSA unter den in (a) angegebenen Bedingungen gibt man zum o-Xylol, bevor es über den Katalysator geleitet wird, ohne Unterbrechung der Oxidation 1 g Triphenylphosphin. Die Konzentration an Triphenylphosphin im o-Xylol beträgt 0,5 Gew.-%.

Anschließend wird die Zufuhr von o-Xylol zum Reaktor unterbrochen und der Katalysator bei einer Temperatur der Salzschmelze von 394°C 32 Stunden unter eine Atmosphäre eines Gemisches aus Luft und $SO_2$ im Volumenverhältnis 5 : 1 gestellt.

Nach erneuter Inbetriebnahme des Reaktors durch Einleiten von 4 Nm³ Luft/h, die mit 60 g o-Xylol/Nm³ beladen ist, kann die Temperatur der Salzschmelze von 394 auf 388°C gesenkt werden, ohne daß der Phthalidgehalt im erzeugten PSA über 0,03% ansteigt.

Nach einer Betriebszeit von 2 Tagen führt man abermals die oben beschriebene 32stündige Behandlung des Katalysators mit dem Gemisch aus Luft und $SO_2$ durch. Nach Inbetriebnahme des Reaktors unter obigen Bedingungen kann die Temperatur der Salzschmelze auf 378°C gesenkt werden. Der Phthalidgehalt im erzeugten PSA ist kleiner als 0,03 Gew.-% und die Ausbeute an PSA ist auf 110 Gew.-% gestiegen.

9 Monate nach der in diesem Beispiel beschriebenen Aktivierung des Katalysators ist eine Erhöhung der Temperatur der Salzschmelze noch nicht notwendig.

Der Phthalidgehalt im erzeugten PSA liegt unter 0,03 Gew.-% und die Ausbeute an PSA beträgt unverändert 110 Gew.-%.

**Patentansprüche**

1. Verfahren zur Reaktivierung von Vanadium enthaltenden Trägerkatalysatoren, die für die Oxidation von o-Xylol oder Naphthalin zu Phthalsäureanhydrid verwendet werden, dadurch gekennzeichnet, daß man den Trägerkatalysator, der aus einem inerten Träger und der darauf aufgebrachten aktiven Masse besteht, wobei die aktive Masse 1 bis 30 Gew.-% Vanadiumpentoxid, 70 bis 99 Gew.-% Titandioxid und bis zu 5 Gew.-% Oxide der Elemente Rubidium, Caesium, Phosphor oder Antimon enthält, zuerst bei Temperaturen von 100 bis 600°C in Gegenwart eines Sauerstoff enthaltenden Gases mit einer flüchtigen Phosphorverbindung und dann bei Temperaturen von 50 bis 600°C mit Schwefeltrioxid oder einem Gemisch aus einer flüchtigen Schwefelverbindung und einem Sauerstoff enthaltenden Gas behandelt.

2. Verfahren nach Anspruch 1, dadurch gekennzeichnet, daß man als flüchtige Phosphorverbindung Triphenylphosphin verwendet.

3. Verfahren nach Anspruch 1, dadurch gekennzeichnet, daß man als flüchtige Schwefelverbindung Schwefeldioxid verwendet.

4. Verfahren nach Anspruch 1, dadurch gekennzeichnet, daß man bei der Herstellung von Phthalsäureanhydrid durch Oxidation von o-Xylol an dem Vanadium enthaltenden Trägerkatalysator bei Temperaturen von 300 bis 500° C die flüchtige Phosphorverbindung zusammen mit dem o-Xylol und dem Sauerstoff enthaltenden Gas über den Katalysator leitet.

## Claims

1. A process for reactivating a supported vanadium-containing catalyst which is used for the oxidation of o-xylene or naphthalene to phthalic anhydride, wherein the supported catalyst, consisting of an inert carrier and the active material applied thereto, which active material contains from 1 to 30% by weight of vanadium pentoxide, from 70 to 99% by weight of titanium dioxide and up to 5% by weight of oxides of rubidium, cesium, phosphorus or antimony, is treated first at from 100 to 600°C, with a volatile phosphorus compound in the presence of an oxygen-containing gas, and then at from 50 to 600°C with sulfur trioxide or a mixture of a volatile sulfur compound and an oxygen-containing gas.

2. A process as claimed in claim 1, wherein triphenylphosphine is used as the volatile phosphorus compound.

3. A process as claimed in claim 1, wherein sulfur dioxide is used as the volatile sulfur compound.

4. A process as claimed in claim 1, wherein, in the preparation of phthalic anhydride by the oxidation of oxylene over the supported vanadium-containing catalyst at from 300 to 500° C, the volatile phosphorus compound is passed, together with the o-xylene and the oxygen-containing gas, over the catalyst.

## Revendications

1. Procédé pour réactiver des catalyseurs au vanadium sur support, utilisés pour l'oxydation de l'orthoxylène ou du naphtalène en anhydride phtalique, caractérisé en ce que le catalyseur sur support, constitué d'une matière active déposée sur un support inerte, la matière active contenant entre 1 et 30% en poids de pentoxyde de vanadium, entre 70 et 99% en poids de bioxyde de titane et jusqu'à 5% en poids d'oxydes des éléments rubidium, césium, phosphore ou antimoine, est traité d'abord, entre 100 et 600°C et en présence d'un gaz renfermant de l'oxygène, avec un dérivé volatil du phosphore, et ensuite, entre 50 et 600°C, avec l'anhydride sulfurique ou avec un mélange d'un dérivé volatil du soufre et d'un gaz renfermant de l'oxygène.

2. Procédé suivant la revendication 1, caractérisé en ce que le dérivé volatil du phosphore est la triphényl-phosphine.

3. Procédé suivant la revendication 1, caractérisé en ce que le dérivé volatil du soufre est l'anhydride sulfureux.

4. Procédé suivant la revendication 1, caractérisé en ce que, lors de la préparation de l'anhydride phtalique par oxydation de l'ortho-xylène entre 300 to 500°C sur le catalyseur au vanadium sur support, on fait passer le dérivé volatil du phosphore avec l'o-xylène et le gaz renfermant de l'oxygène sur le catalyseur.